# EUROPÄISCHE PATENTSCHRIFT

(11) **EP 0 874 832 B1**
(45) Veröffentlichungstag und Bekanntmachung des Hinweises auf die Patenterteilung: **10.04.2002**
(21) Anmeldenummer: 96942322.7
(22) Anmeldetag: 04.12.1996
(51) Int. Cl.: C07D 251/60, C07D 251/62

(54) **VERFAHREN ZUR HERSTELLUNG VON REINEM MELAMIN**
PROCESS FOR PRODUCING PURE MELAMINE
PROCEDE POUR PRODUIRE DE LA MELAMINE PURE

(30) Priorität: 07.12.1995 AT 199495
(43) Veröffentlichungstag der Anmeldung: 04.11.1998
(73) Patentinhaber: Agrolinz Melamin GmbH, A-4021 Linz (AT)
(72) Erfinder: CANZI, Lorenzo, I-20131 Mailand (IT); COUFAL, Gerhard, A-4060 Leonding (AT); MÜLLNER, Martin, A-4020 Linz (AT)
(74) Vertreter: VA TECH Patente GmbH & Co
(86) Internationale Anmeldenummer: EP9605389
(87) Internationale Veröffentlichungsnummer: WO9720826

(56) Entgegenhaltungen:
- WO-A-96/20182
- WO-A-96/23778
- US-A- 3 116 294
- US-A- 3 637 686
- US-A- 4 565 867
- US-A- 5 514 796
- US-A- 5 514 797

## Beschreibung

Aus der Literatur ist bereits eine Vielzahl von Verfahren zur Herstellung von Melamin bekannt. Ein bevorzugtes Ausgangsmaterial ist dabei Harnstoff, der entweder bei hohem Druck und nichtkatalytisch oder bei niedrigem Druck und unter Verwendung eines Katalysators zu Melamin, Ammoniak und CO₂ umgesetzt wird.

Die bekannten Hochdruckverfahren, etwa nach Melamine Chemicals, Montedison oder Nissan, bei denen das Melamin zuerst als Flüssigkeit gebildet wird, haben zwar einen im Vergleich zu Niederdruckverfahren geringeren Energieverbrauch, Melamin enthält jedoch, falls keine Reinigungsstufen vorhanden sind, Verunreinigungen wie Melam, Melem, Ammelin, Ammelid oder Ureidomelamin, die bei manchen Weiterverarbeitungen des Melamins stören.

Die Aufarbeitung des durch ein Hochdruckverfahren hergestellten Melamins erfolgt beispielsweise nach US 4 565 867 (Melamine Chemicals) durch Abtrennung der CO₂- und NH₃-Abgase vom flüssigen Melamin, wobei der Druck und die Temperatur bevorzugt auf den gleichen Werten, wie sie im Reaktor vorliegen, gehalten werden. Anschließend wird das flüssige Melamin einer Produktkühleinheit zugeführt, worin von 105 bis 175 bar auf etwa 14 bis 42 bar entspannt und gleichzeitig mit flüssigem Ammoniak rasch von 350 bis 430°C auf 48 bis 110°C gekühlt bzw. abgeschreckt wird (Quenchen), wodurch sich Melamin als festes Produkt abscheidet.

Gemäß US 3,116,294 (Montecatini) werden ebenfalls zuerst die CO₂- und NH₃-Abgase abgetrennt, das flüssige Melamin wird, um noch gelöstes CO₂ zu entfernen, im Gegenstrom mit NH₃ behandelt, in einem weiteren Reaktor gesammelt und eine bestimmte Zeit darin verweilen gelassen. Zuletzt wird Melamin aus dem zweiten Reaktor entnommen und durch Abschrecken mit Wasser oder durch Mischen mit kalten Gasen rasch abgekühlt.

Die Reinheit von Melamin, das durch eines dieser Verfahren gewonnenen wird, ist jedoch für viele Anwendungen, etwa bei der Herstellung von Melamin-Formaldehydharzen für Oberflächenbeschichtungen, nicht ausreichend, da insbesondere der Gehalt an Melem zu hoch ist.

Gemäß US 3,637,686 (Nissan) wird die durch thermische Zersetzung von Harnstoff erhaltene rohe Melaminschmelze schnell mit flüssigem NH₃ oder kaltem NH₃-Gas auf 200 bis 270°C abgekühlt, und in einem zweiten Schritt mit wäßriger NH₃-Lösung auf 100 bis 200°C weiter abgekühlt. Anschließend muß, um eine zufriedenstellende Reinheit des Melamins zu erreichen, umkristallisiert werden.

Aufgabe der vorliegenden Erfindung war es demnach, ein Verfahren zu finden, das die Herstellung von reinem Melamin mit einer Reinheit von bis zu über 99,8 % mit einem deutlich reduzierten Gehalt an Verunreinigungen, insbesondere an Melem und Melam ermöglicht.

Unerwarteterweise konnte diese Aufgabe durch ein Verfahren gelöst werden, bei welchem flüssiges, ammoniakhältiges Melamin bei einer Temperatur beim oder knapp über dem vom jeweils herrschenden Ammoniakpartialdruck abhängigen Festpunkt von Melamin rasch entspannt wird, wobei der Festpunkt je nach Temperatur bei Entspannungsbeginn und gewünschtem Enddruck um etwa bis zu 60°C ansteigt, und sich festes Melamin abscheidet.

Gegenstand der vorliegenden Erfindung ist demnach ein Verfahren zur Herstellung von reinem Melamin, das dadurch gekennzeichnet ist, daß flüssiges ammoniakgesättigtes Melamin bei einer Temperatur, die um 0 - 60 °C über dem vom jeweils herrschenden Ammoniakpartialdruck abhängigen Festpunkt des Melamins, jedoch unter 350 °C liegt, wobei höhere Drücke einen größeren Abstand vom Festpunkt des Melamins erlauben als niedrigere Drücke, von einem Ammoniakpartialdruck p₁ zwischen 400 und 100 bar auf einen Ammoniakpartialdruck p₂ zwischen 200 und 6 bar rasch entspannt wird, wodurch sich reines Melamin in fester Form abscheidet, worauf in beliebiger Reihenfolge auf Atmosphärendruck entspannt, auf Raumtemperatur gekühlt und das reine Melamin isoliert wird.

Das erfindungsgemäße Verfahren eignet sich zur Reinigung von Melamin, das in einem beliebigen, etwa aus dem Stand der Technik bekannten Prozeß anfällt und insbesondere Verunreinigungen wie Melem und Melam enthält, wobei das Melamin entweder als Schmelze bzw. in der Flüssigphase oder in kristalliner Form vorliegen kann.

Liegt das zu reinigende Melamin bereits als Schmelze bzw. als Flüssigphase, wie beispielsweise im Anschluß an einen Hochdruckreaktor zur Melaminsynthese durch Harnstoffumsetzung vor, so wird der Druck und die Temperatur der Schmelze bzw. des flüssigen Melamins auf den für die Entspannung gewünschten Anfangs-Ammoniakpartialdruck zwischen etwa 400 und 50 bar, bevorzugt zwischen etwa 400 und 80 bar, besonders bevorzugt zwischen etwa 300 und 100 bar und auf den entsprechenden oben definierten Temperaturwert, d.h. auf eine Temperatur, die um etwa 0 bis 60°C, bevorzugt um etwa O bis 40°C, besonders bevorzugt um etwa 0 bis 20 °C über dem vom jeweils herrschenden Ammoniakpartialdruck abhängigen Festpunkt des Melamins liegt, gebracht. Dabei ist zu beachten, daß bei höheren Drücken die Temperaturdifferenz zwischen Festpunkt des Melamins und der einzustellenden Temperatur bei Entspannungsbeginn größer sein kann als bei niederen Drücken, da der Festpunkt der Schmelze bei höheren Drücken bei niedrigeren Temperaturen liegt als bei niedrigen Drücken. Um den für die Entspannung gewünschten Temperaturwert zu erreichen, wird die Temperatur, falls erforderlich, abgesenkt. Besonders bevorzugt liegt diese Temperatur bei unter etwa 350 °C. Die Abkühlung kann dabei sowohl schnell als auch langsam durchgeführt werden. Bevorzugt erfolgt dies langsam mit einer Kühlrate von 0.8 bis 10°C/min. Da die Melaminschmeize bei niedrigerer Temperatur mehr Ammoniak aufnehmen kann, wird dabei bevorzugterweise Ammoniak zugeführt. Besonders vorteilhaft ist es, die Entspannung des flüssigen, ammoniakhältigen Melamins möglichst nahe bei oder über dem vom jeweiligen Ammoniakpartialdruck abhängigen Festpunkt des Melamins durchzuführen. Mit dem vorliegenden Verfahren ist es weiters möglich, festes, verunreinigtes Melamin zu reinigen. Das zu reinigende Melamin, das in kristalliner Form oder als Pulver vorliegt, wird zuerst bei einem Ammoniakpartialdruck zwischen etwa 400 und 50 bar, bevorzugt zwischen etwa 400 und 80 bar, besonders bevorzugt zwischen etwa 300 und 100 bar auf eine Temperatur, die um etwa 0 bis 60°C, bevorzugt um etwa 0 bis 40°C, besonders bevorzugt um etwa 0 bis 20 °C über dem vom jeweils herrschenden Ammoniakpartialdruck abhängigen Festpunkt des Melamins, liegt, erwärmt. Zum sicheren Aufschmelzen von festem Melamin ist es zweckmäßig , zuerst auf bis etwa 370 °C zu erwärmen und dann auf die gewünschte Entspannungstemperatur abzukühlen, um sicherzustellen, daß das Melamin vollständig geschmolzen ist. Bevorzugt liegt die gewünschte Entspannungstemperatur bei unter etwa 350 °C.

Wiederum ist zu beachten, daß die Temperaturdifferenz bei höheren Drücken größer sein kann als bei niedrigeren Drücken.

Bevorzugt wird das erfindungsgemäße Verfahren unmittelbar anschließend an ein Melamin-Hochdruckverfahren durchgeführt. Beispiele für Hochdruckverfahren sind etwa der Melamine Chemical-, Montedison- oder Nissanprozeß, wie sie beispielsweise in Ullmann's Encyclopedia of Industrial Chemistry, 5^{th} Edition, Vol. A16, pp 174-179 beschrieben sind. Die Harnstoffumsetzung erfolgt nach diesen Verfahren zumeist in einem Temperaturbereich von etwa 370 bis 430°C und einem Druck von etwa 70 bis 300 bar. Das dabei entstehende Melamin wird schließlich als flüssige Phase erhalten.
Nach dem erfindungsgemäßen Verfahren wird, falls erforderlich, der für die rasche Entspannung gewünschte Anfangsammoniakpartialdruck zwischen etwa 400 und 50 bar eingestellt. Um die entsprechende Ausgangstemperatur für die Entspannung einzustellen, wird das aus dem Harnstoffumsetzungsprozeß erhaltene flüssige Melamin von der im Reaktor herrschenden Temperatur mittels geeigneter Kühlvorrichtungen, etwa mittels Wärmetauscher, auf den entsprechenden Wert, d.h. auf eine Temperatur, die um etwa 0 bis 60°C, bevorzugt um etwa 0 bis 40 °C, besonders bevorzugt um etwa 0 bis 20 °C über dem vom jeweils eingestellten Ammoniakpartialdruck abhängigen Festpunkt des Melamins liegt, abgekühlt. Die Abkühlung kann dabei auf beliebige Weise sowohl schnell als auch langsam erfolgen. Bevorzugt erfolgt die Abkühlung mit einer Geschwindigkeit die zwischen etwa 0,8 °C/min und 10°C/min liegt, bevorzugt unter weiterer Ammonaikzufuhr. Die Temperatur kann auch mittels eines Kühlprogrammes erniedrigt werden, wobei beispielsweise Kühl- und Haltephasen oder unterschiedliche Kühlraten sich abwechseln können.
Vor der Abkühlung wird das bei der Reaktion entstehende NH₃/CO₂-Gasgemisch vom flüssigen Melamin abgetrennt und das im flüssigen Melamin gelöste CO₂ durch Einbringen von gasförmigem Ammoniak reduziert. Weiters ist es möglich, vor dem Entspannen das flüssige Melamin für etwa 5 Minuten bis zu 20 Stunden bei dem eingestellten Ammoniakpartialdruck verweilen zu lassen. Bevorzugt wird zwischen 10 Minuten und 10 Stunden, besonders bevorzugt zwischen 30 Minuten und 4 Stunden verweilen gelassen. Längere Verweilzeiten sind gewünschtenfalls auch möglich.
Das zu reinigende ammoniakhältige Melamin liegt vor dem Entspannen in flüssiger Form vor. Beim Entspannen wird der Druck in Abhängigkeit vom eingestellen Anfangsdruck auf einen Wert zwischen Atmosphärendruck und etwa 200 bar, bevorzugt auf zwischen Atmosphärendruck und etwa 150 bar, besonders bevorzugt auf zwischen Atmosphärendruck und etwa 50 bar rasch abgesenkt.
Dabei entweicht das im Melamin gelöste Ammoniak, wodurch sich der Festpunkt des nunmehr weitgehend von Ammoniak befreiten Melamins um bis zu etwa 60°C erhöht, sodaß sich das flüssige Melamin sofort verfestigt, und die Bildung von Nebenprodukten, insbesondere von Melem verhindert wird. Durch die Entspannung sinkt einerseits die Temperatur im System, andererseits wird durch die Verfestigung des Melamins Kristallisationswärme frei. Es wird angenommen, daß der Prozeß in Summe etwa autotherm, verläuft.
Es ist vorteilhaft, wenn die Melaminschmelze vor dem Entspannen mit Ammoniak gesättigt ist. Es ist jedoch auch möglich, die Entspannung mit einer nicht mit Ammoniak gesättigten Melaminschmelze durchzuführen, wobei der Vorteil der Schmelzpunkterhöhung jedoch nicht voll ausgenützt werden kann.

Die Entspannung kann direkt in dem Behälter bzw. in der Apparatur, in die das flüssige Melamin eingebracht wurde, erfolgen. Die Entspannung kann jedoch auch durch Überführung bzw. Einsprühen in einen oder mehrere weitere Behälter mittels geeigneter Sprühvorrichtungen durchgeführt werden. Bevorzugt ist dabei in den Behältern eine Ammoniakatmosphäre vorhanden. Weiters ist es besonders vorteilhaft, in einen Behälter zu entspannen, in dem etwa die gleiche Temperatur herrscht, wie in dem Gefäß, aus dem entspannt wird.
Das nun feste Melamin kann gewünschtenfalls noch für einige Zeit, etwa für 1 Minute bis 20 Stunden bei dem nun herrschenden Ammoniakpartialdruck und den herrschenden Temperaturen gehalten werden. Bevorzugt wird das feste Melamin zwischen 10 Minuten und 10 Stunden, besonders bevorzugt zwischen 30 Minuten und 3 Stunden unter diesen Bedingungen verweilen gelassen. Bevorzugt sollte dabei die Temperatur unter etwa 290°C betragen. Besonders bevorzugt läßt man das nunmehr feste Melamin bei einer Temperatur zwischen etwa 280 und 250°C verweilen, wobei die Temperatur während dieses Zeitraumes sowohl konstant gehalten oder kontinuierlich oder diskontinuierlich verändert werden kann. Im Anschluß an diesen Entspannungsvorgang bzw. an das Verweilenlassen kann das nunmehr feste Melamin auf beliebige Weise und je nach den technischen Gegebenheiten, zuerst auf Raumtemperatur abgekühlt und dann auf Atmosphärendruck weiter entspannt oder gleichzeitig oder in umgekehrter Reihenfolge weiter entspannt und abgekühlt werden. Bevorzugt wird zuerst weiter entspannt und im Anschluß daran auf Raumtemperatur abgekühlt.
Das Abkühlen des bereits festen Melamins auf Raumtemperatur erfolgt beispielsweise durch Abschrecken mit einem kalten, flüssigen Medium, etwa mittels flüssigem Ammoniak, durch Mischen mit kalten Gasen, durch Abkühlen mittels Wärmetauscher beispielsweise mittels eines Temperaturprogrammes, oder durch einfaches Entfernen des Heizmediums.
Das erfindungsgemäße Verfahren kann je nach Bedarf sowohl in einem diskontinuierlichen als auch in einem kontinuierlichen Verfahren durchgeführt werden. Besonders vorteilhaft ist es, das erfindungsgemäße Verfahren kontinuierlich durchzuführen.
In einer vorteilhaften Ausführungsform wird nach Abtrennen von NH₃ und CO₂ die Melaminschmelze bei einem Ammoniakdruck von etwa 70 - 300 bar, bevorzugt beim herrschenden Reaktordruck, verweilengelassen, die Temperatur unter weiterer Ammoniakzufuhr möglichst nahe an den bei diesem Ammoniakpartialdruck herrschenden Festpunkt abgesenkt, dann auf etwa 50 bar bis Atmosphärendruck entspannt, gegebenenfalls verweilengelassen und weiter entspannt und auf Raumtemperatur gekühlt.

Die einzelnen Schritte des erfindungsgemäßen Verfahrens, wie
- gegebenenfalls Abtrennen eines NH₃/CO₂-Gasgemisches mit
- gegebenenfalls anschließendem Reduzieren des gelösten CO₂-Gehaltes
- gegebenenfalls Verweilenlassen und Abkühlen auf Entspannungstemperatur
- Entspannen
- gegebenenfalls Verweilenlassen im Festzustand
- gegebenenfalls weiteres Entspannen auf Atmosphärendruck
   Abkühlen auf Raumtemperatur
können dabei beispielsweise in getrennten, für den jeweiligen Schritt geeigneten Behältern bzw. Apparaturen durchgeführt werden. Es ist jedoch auch möglich, 2 oder mehrere dieser Schritte in gemeinsamen Apparaturen durchzuführen. Die Prozeßführung muß jedoch den jeweiligen Gegebenheiten angepaßt werden.

Um die Abhängigkeit des Festpunktes von Melamin vom herrschenden Ammoniakpartialdruck zu ermitteln, wurden entsprechende Abkühlversuche durchgeführt.

Melamin wird durch das erfindungsgemäße Verfahren in kristalliner Form bzw. als Pulver mit einer Reinheit von bis zu über 99,8 % erhalten und weist einen deutlich reduzierten Gehalt insbesondere an Melem und Melam auf.

### Beispiel 1-6:

Bestimmung des vom Ammoniakpartialdruck abhängigen Festpunktes von Melamin.

9,9 g Melamin mit 0,1 g Melem wurden mit der für die Einstellung eines bestimmten Druckes p nötigen Ammoniakmenge in einen Autoklaven eingewogen und aufgeschmolzen. Das Reaktionsgemisch wurde für einige Stunden h bei 370°C verweilen gelassen, um die Gleichgewichtseinstellung zu ermöglichen. Danach wurde das Reaktionsgemisch abkühlen gelassen und der Temperaturverlauf kontrolliert, wobei der Festpunkt durch einen kurzen Temperaturanstieg erkennbar war. Die Verfahrensparameter wie Druck, Verweilzeit sowie der ermittelte Festpunkt (Fp) sind aus Tabelle 1 ersichtlich. Die Abhängigkeit des Festpunktes von Melamin vom jeweils herrschenden Ammoniakpartialdruck ist in Fig. 1 dargestellt.

**Tabelle 1:**

| Bsp. | p (bar) | h | Fp (°C) |
|---|---|---|---|
| 1 | 350 | 6 | 294 |
| 2 | 300 | 6 | 300 |
| 3 | 250 | 6 | 306 |
| 4 | 200 | 6 | 317 |
| 5 | 150 | 6 | 328 |
| 6 | 110 | 6 | 331 |

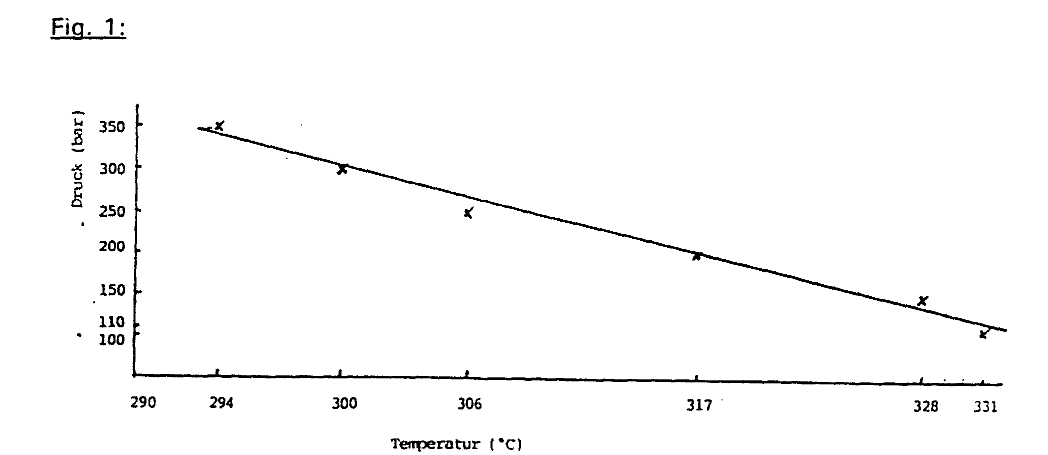

### Beispiel 7-19:

In einem Laborautoklaven mit 70 ml Volumen wurden 9,9 g Melamin mit einem Melamgehalt von 1300 ppm und 0,1 g Melem, sowie die zur Erzielung des vor der Entspannung gewünschten Druckes p₁ nötige Ammoniakmenge eingebracht. Anschließend wurde der Autoklav auf eine Temperatur T₁ gebracht, gegebenenfalls in x Minuten auf eine Temperatur T₂ abgekühlt und t₁ Minuten auf dieser Temperatur gehalten. Danach wurde rasch auf einen bestimmten Druck p₂ entspannt und gegebenenfalls anschließend t₂ Minuten unter den nun herrschenden Reaktionsbedingungen gehalten.
Nach Beendigung dieses Vorgangs wurde schlagartig im Wasserbad abgekühlt und entspannt und das erhaltene Melamin analysiert.
Die Verfahrensparameter wie Druck p₁ und p₂, Temperatur T₁ und T₂, Abkühlzeit von T₁ auf T₂ in x Minuten, Verweilzeiten t₁ und t₂, sowie der Endgehalt an Melem (ME) und Melam (MA) sind aus Tabelle 2 ersichtlich.

**Tabelle 2:**

| Bsp. | p₁ (bar) | T₁ (°C) | x (min) | T₂ (°C) | t₁ (min) | p₂ (bar) | t₂ (min) | ME ppm | MA ppm |
|---|---|---|---|---|---|---|---|---|---|
| 7 | 300 | 310 | 0 | 310 | 120 | 150 | 0 | 40 | <300 |
| 8 | 250 | 320 | 0 | 320 | 120 | 150 | 0 | 65 | 350 |
| 9 | 250 | 370 | 60 | 320 | 120 | 35 | 0 | 190 | 400 |
| 10 | 250 | 370 | 60 | 320 | 120 | 50 | 5 | 80 | 410 |
| 11 | 250 | 370 | 60 | 320 | 120 | 150 | 5 | 80 | 500 |
| 12 | 250 | 370 | 60 | 320 | 120 | 150 | 5 | 45 | 310 |
| 13 | 250 | 370 | 60 | 320 | 30 | 150 | 5 | 25 | <300 |
| 14 | 250 | 370 | 60 | 320 | 10 | 50 | 5 | 65 | <300 |
| 15 | 250 | 370 | 30 | 320 | 10 | 50 | 5 | 185 | 530 |
| 16 | 250 | 370 | 60 | 320 | 10 | 150 | 5 | 50 | <300 |
| 17 | 250 | 370 | 30 | 320 | 10 | 150 | 5 | 50 | <300 |
| 18 | 250 | 370 | 7 | 320 | 10 | 150 | 5 | 45 | <300 |
| 19 | 200 | 335 | 0 | 335 | 120 | 150 | 0 | 220 | 440 |

### Beispiel 20-36:

In einem Laborautoklaven A1 mit 100 ml Volumen wurden x g Melamin (M₀) mit einem Melamgehalt (MA₀) von 1300 ppm und y g Melem (ME₀), sowie die zur Erzielung des vor der Entspannung gewünschten Druckes p1 nötige Ammoniakmenge eingebracht. Anschließend wurde der Autoklav auf eine Temperatur von 370°C (T₁) gebracht und t₁ Minuten auf T₁ gehalten. Anschließend wurde in z₁ Minuten auf eine Temperatur T₂ abgekühlt und t₂ Minuten auf dieser Temperatur gehalten.

In Beispiel 20-32 wurde im Anschluß daran das in A1 befindliche Melamin in einen Laborautoklaven A2 mit 1000 ml Volumen, der auf einer Temperatur T₃ und bei einem Druck p₃ gehalten wurde, gesprüht.
In Beispiel 33 und 34 wurde im Autoklaven A1 die Temperatur T₂ in t_{2S} Minuten auf die Temperatur T_{2S} abgesenkt. Gleichzeitig dazu wurde im Autoklaven A2 die Temperatur T₃ auf die Temperatur T_{2S} und der Druck auf den Wert von p₃ eingestellt und das Melamin von A₁ in A₂ gesprüht.
In Beispiel 35 und 36 wurde aus dem Autoklaven A1 nur ein Teil des flüssigen Melamins in den Autoklaven A2 gesprüht, indem ein Ventil in der Leitung zwischen A1 und A2 kurz geöffnet und wieder geschlossen wurde. Dadurch wurde der Druckabfall in A1 und der Druckanstieg in A2 gering gehalten.

Dabei änderte sich in A1 nach dem Produkttransfer die Temperatur T₂ auf einen Wert T_{2.1}, der Druck p₁ auf einen Wert von p₂. Im Autoklaven A2 änderte sich die Temperatur T₃ auf einen Wert T_{3.1} und der Druck p₃ auf einen Wert p_{3.1}.
Das in A1 verbleibende Melamin (M₁) wurde auf eine Temperatur T₄ in z₂ Minuten abgekühlt, anschließend entspannt, rasch abgekühlt und analysiert (ME₁, MA₁)
Das in A2 gesprühte Melamin (M₂) wurde auf eine Temperatur T₅ in z₃ Minuten abgekühlt, entspannt, rasch abgekühlt und analysiert (ME₂, MA₂).
Die Verfahrensparameter wie Druck p₁, p₂, p₃ und p_{3.1}, Temperatur T₁, T₂,T_{2.1}, T_{2S} T₃, T_{3.1}, T₄ und T₅, Abkühlzeit z₁, z₂ und z₃ Minuten, Verweilzeit t₁, t₂ und t_{2S}, sowie die Einwaage an Melamin (M₀), die Auswaagen an Melamin (M₁, M₂), sowie der Anfangsgehalt an Melem (ME₀) und der Endgehalt an Melem (ME₁, ME₂) und Melam (MA₁, MA₂) sind aus Tabelle 3 ersichtlich.

**Tabelle 3:**

| Autoklav A1 (T₁ = 370°C) vor Produkttransfer | | | | | | | | | |
|---|---|---|---|---|---|---|---|---|---|
| Bsp. | xM₀ (g) | yME₀ (g) | p₁ (bar) | t₁ (min) | z₁ (min) | T₂ (°C) | t₂ (min) | T_{2S} (°C) | t_{2S} (min) |
| 20 | 9,9 | 0.1 | 250 | 0 | 60 | 320 | 10 | | |
| 21 | 9,9 | 0,1 | 250 | 0 | 60 | 315 | 10 | | |
| 22 | 9,9 | 0,1 | 250 | 0 | 60 | 310 | 10 | | |
| 23 | 9,9 | 0,1 | 350 | 0 | 60 | 300 | 10 | | |
| 24 | 29,7 | 0,3 | 250 | 90 | 60 | 320 | 10 | | |
| 25 | 19,8 | 0,2 | 250 | 120 | 60 | 320 | 10 | | |
| 26 | 19,8 | 0,2 | 250 | 120 | 60 | 320 | 10 | | |
| 27 | 9,9 | 0,1 | 300 | 0 | 60 | 315 | 10 | | |
| 28 | 9,9 | 0,1 | 200 | 0 | 60 | 330 | 10 | | |
| 29 | 9,9 | 0,1 | 350 | 0 | 60 | 303 | 10 | | |
| 30 | 9,9 | 0,1 | 350 | 0 | 60 | 310 | 10 | | |
| 31 | 9,9 | 0,1 | 200 | 60 | 60 | 330 | 10 | | |
| 32 | 19,8 | 0,2 | 250 | 120 | 60 | 320 | 10 | | |
| 33 | 9,9 | 0,1 | 250 | 60 | 53 | 320 | 120 | 312 | 24 |
| 34 | 9,9 | 0,1 | 250 | 60 | 41 | 330 | 120 | 314 | 32 |
| 35 | 9,9 | 0,1 | 265 | 120 | 69 | 316 | 0 | | |
| 36 | 9,9 | 0,1 | 260 | 120 | 59 | 317 | 0 | | |
| | | | | | | | | | |

| Autoklav A1 nach Produkttransfer | | | | | | | | | |
|---|---|---|---|---|---|---|---|---|---|
| Bsp. | T_{2.1} (°C) | p_{2.} (bar) | M₁ (g) | T₄ (°C) | z₂ (min) | ME₁ (ppm) | MA₁ (ppm) | | |
| 20 | 307 | 90 | 5,5 | 245 | 13 | 20 | <300 | | |
| 21 | 285 | 85 | 7,0 | RT | r | 20 | <300 | | |
| 22 | 275 | 85 | 8,0 | 250 | 14 | 20 | <300 | | |
| 23 | 270 | 50 | 4,0 | 250 | 4 | <20 | <300 | | |
| 24 | 326 | 175 | 22,0 | 280 | 14 | <20 | <300 | | |
| 25 | 304 | 70 | 1,0 | 280 | 6 | 55 | 490 | | |
| 26 | 307 | 80 | 10,5 | 280 | 13 | 20 | <300 | | |
| 27 | 294 | 80 | 3,0 | 280 | 12 | 25 | <300 | | |
| 28 | 314 | 80 | 1,2 | 280 | 18 | <20 | 380 | | |
| 29 | 274 | 60 | 3,5 | 250 | 8 | <20 | 370 | | |
| 30 | 275 | 65 | 1,5 | 250 | 4 | <20 | <300 | | |
| 31 | 306 | 50 | 1,5 | 280 | 8 | 100 | 800 | | |
| 32 | 302 | 65 | 1,0 | 280 | 10 | 50 | 630 | | |
| 33 | 292 | 80 | 4,9 | 300 | 10 | <20 | <300 | | |
| 34 | 295 | 80 | 0,8 | 300 | 6 | <20 | <300 | | |
| 35 | 311 | 220 | 3,8 | 300 | 6 | <50 | 800 | | |
| 36 | - | 235 | 3,2 | 300 | 6 | <50 | 820 | | |
| | | | | | | | | | |

| Autoklav A2 | | | | | | | | | |
|---|---|---|---|---|---|---|---|---|---|
| Bsp | T₃ (°C) | p₃ (bar) | T_{3.1} (°C) | p_{3.1} (bar) | M₂ (g) | T₅ (°C) | z₃ (min) | ME₂ (ppm) | MA₂ (ppm) |
| 20 | 277 | 52 | 284 | 79 | 3,5 | 250 | 12 | 75 | <300 |
| 21 | 280 | 51 | 282 | 76 | 2,7 | RT | r | 75 | 600 |
| 22 | 281 | 52 | 282 | 76 | 1,1 | 250 | 8 | 55 | 650 |
| 23 | 280 | 0 | 280 | 40 | 6,0 | 250 | 12 | 60 | 1100 |
| 24 | 320 | 6 | 320 | 15 | 3,0 | 280 | 15 | 40 | 1600 |
| 25 | 300 | 40 | 309 | 68 | 15,5 | 280 | 8 | 95 | 360 |
| 26 | 302 | 50 | 306 | 74 | 8,0 | 280 | 11 | 70 | 540 |
| 27 | 282 | 40 | 285 | 72 | 4,5 | 280 | 4 | 20 | 780 |
| 28 | 302 | 50 | 304 | 72 | 3,8 | 280 | 12 | 65 | 650 |
| 29 | 280 | 17 | 280 | 60 | 5,5 | 250 | 10 | 20 | 1400 |
| 30 | 300 | 20 | 300 | 62 | 4,5 | 280 | 9 | 25 | 770 |
| 31 | 298 | 20 | 300 | 48 | 6,5 | 280 | 12 | 110 | 1000 |
| 32 | 300 | 30 | 305 | 62 | 16,0 | 280 | 11 | 45 | 790 |
| 33 | 312 | 52 | 312 | 78 | 2,9 | 280 | 15 | <20 | <300 |
| 34 | 314 | 51 | 314 | 76 | 6,2 | 280 | 15 | 20 | 300 |
| 35 | 316 | 53 | 316 | 57 | 2,8 | 280 | 15 | <20 | 400 |
| 36 | 280 | 55 | 280 | - | 3,2 | 275 | 3 | 40 | 750 |
| RT auf Raumtemperatur r rasch | | | | | | | | | |

## Patentansprüche

1. Verfahren zur Herstellung von reinem Melamin, **dadurch gekennzeichnet**, das flüssiges ammoniakgesättigtes Melamin bei einer Temperatur, die um 0 - 60°C über dem vom jeweils herrschenden Ammoniakpartialdruck abhängigen Festpunkt des Melamins, jedoch unter 350°C liegt, wobei höhere Drücke einen größeren Abstand vom Festpunkt des Melamins erlauben als niedrigere Drücke, von einem Ammoniakpartialdruck p₁ zwischen 400 und 100 bar auf einen Ammoniakpartialdruck p₂ zwischen 200 und 6 bar rasch entspannt wird, wodurch sich reines Melamin in fester Form abscheidet, worauf in beliebiger Reihenfolge auf Atmosphärendruck entspannt, auf Raumtemperatur gekühlt und das reine Melamin isoliert wird.

2. Verfahren nach Anspruch1, **dadurch gekennzeichnet, dass** das flüssige Melamin von einem Ammoniakpartialdruck p₁ zwischen 400 und 200 bar rasch entspannt wird.

3. Verfahren nach einem der Ansprüche 1 oder 2, **dadurch gekennzeichnet, dass** das flüssige Melamin bei einer Temperatur, die 0 bis 40°C über dem von jeweiligen Ammoniakpartialdruck abhängigen Festpunkt des Melamins, jedoch unter 350°C liegt, rasch entspannt wird.

4. Verfahren nach Anspruch 3, **dadurch gekennzeichnet, dass** das flüssige Melamin bei einer Temperatur, die um 0 bis 20°C über dem vom jeweiligen Ammoniakpartialdruck abhängigen Festpunkt des Melamins, jedoch unter 350°C liegt, rasch entspannt wird.

5. Verfahren nach einem der Ansprüche 1 bis 4, **dadurch gekennzeichnet, dass** das flüssige Melamin auf einen Ammoniakpartialdruck p₂ zwischen 175 und 6 bar, vorzugsweise 150 und 20 bar rasch entspannt wird.

6. Verfahren nach einem der Ansprüche 1 bis 5, **dadurch gekennzeichnet, dass** das flüssige Melamin vor dem raschen Entspannen 5 Minuten bis zu 20 Stunden, bevorzugt 10 Minuten bis 10 Stunden, besonders bevorzugt 30 Minuten bis 4 Stunden bei dem herrschenden Ammoniakpartialdruck verweilen gelassen wird.

7. Verfahren nach einem der Ansprüche 1 bis 6, **dadurch gekennzeichnet, dass** nach dem raschen Entspannen das feste Melamin für 1 Minute bis 20 Stunden, bevorzugt zwischen 10 Minuten und 10 Stunden, besonders bevorzugt zwischen 30 Minuten und 3 Stunden bei dem herrschenden Ammoniakpartialdruck und der herrschenden Temperatur verweilen gelassen wird.

8. Verfahren nach einem der Ansprüche 1 bis 7, **dadurch gekennzeichnet, dass** das feste Melamin bei einer Temperatur von unter 290°C verweilen gelassen wird.

9. Verfahren nach einem der Ansprüche 1 bis 8, **dadurch gekennzeichnet, dass** das feste Melamin bei einer Temperatur zwischen 280 und 250°C verweilen gelassen wird.

10. Verfahren nach einem der Ansprüche 1 bis 9, **dadurch gekennzeichnet, dass** die Entspannung in dem Behälter, in den das flüssige Melamin eingebracht wurde, erfolgt.

11. Verfahren nach einem der Ansprüche 1 bis 10, **dadurch gekennzeichnet, dass** die Entspannung durch Überführen bzw. durch Einsprühen in einen oder mehrere Behälter erfolgt.

12. Verfahren nach Anspruch 11, **dadurch gekennzeichnet, dass** in diesem(n) Behälter(n) eine Ammoniakatmosphäre vorhanden ist.

13. Verfahren nach einem der Ansprüche 11 oder 12, **dadurch gekennzeichnet, dass** in den Behältern etwa die gleiche Temperatur herrscht, wie in dem Behälter, aus dem entspannt wird.

14. Verfahren nach einem der Ansprüche 1 bis 13, **dadurch gekennzeichnet, dass** das bereits feste Melamin durch Abschrecken mit einem kalten flüssigen Medium wie flüssigem Ammoniak, durch Mischen mit kalten Gasen oder mittels Wärmetauscher auf Raumtemperatur gekühlt wird.

15. Verfahren nach einem der Ansprüche 1 bis 14, **dadurch gekennzeichnet, dass** ein zu reinigendes Melamin, das bereits als Schmelze bzw. Flüssigphase vorliegt, insbesondere im Anschluss an eine nichtkatalytische Melaminsynthese unter Hochdruck durch Umsetzung von Harnstoff mit Ammoniak auf die für die Entspannung gewünschte Temperatur und auf den für die Entspannung gewünschten Ammoniakpartialdruck p₁ gebracht wird.

16. Verfahren nach Anspruch 15, **dadurch gekennzeichnet, dass** das geschmolzene bzw. flüssige Melamin durch Temperaturabsenkung auf die gewünschte Temperatur gebracht wird.

17. Verfahren nach Anspruch 16, **dadurch gekennzeichnet, dass** die Temperatur mit einer Kühlrate von 0,8 bis 10°C/min abgesenkt wird.

18. Verfahren nach einem der Ansprüche 15 bis 17, **dadurch gekennzeichnet, dass** das bei der Harnstoffumsetzung entstehende NH₃/CO₂ Gasgemisch vor dem Abkühlen vom flüssigen Melamin abgetrennt und das im flüssigen Melamin gelöste CO₂ durch Einbringen von gasförmigen Ammoniak reduziert wird.

19. Verfahren nach einem der Ansprüche 1 bis 14, **dadurch gekennzeichnet, dass** das zu reinigende Melamin, das vorerst als Feststoff vorliegt, auf eine zum sicheren Aufschmelzen des Melamins ausreichende Temperatur gebracht wird, worauf die Schmelze auf die für die Entspannung gewünschte Temperatur und auf den für die Entspannung gewünschten Ammoniakpartialdruck p₁ gebracht wird.

20. Verfahren nach einem der Ansprüche 1 bis 19, **dadurch gekennzeichnet, dass** bis zur Einstellung der jeweils gewünschten Temperatur bzw. des gewünschten Drucks Ammoniak zugeführt wird.

21. Verfahren nach einem der Ansprüche 1 bis 20, **dadurch gekennzeichnet, dass** das Verfahren kontinuierlich durchgeführt wird.

## Claims

1. A process for preparing pure melamine, which comprises liquid-ammonia-saturated melamine being rapidly depressurized from an ammonia partial pressure p₁ between 400 and 100 bar to an ammonia partial pressure p₂ between 200 and 6 bar at a temperature which is 0-60°C above the melamine solidification point dependent on the respective prevailing ammonia partial pressure, but is below 350°C, with higher pressures permitting a greater distance from the melamine solidification point than lower pressures, as a result of which pure melamine precipitates out in solid form, whereupon, in any sequence, the product is depressurized to atmospheric pressure, cooled to room temperature and the pure melamine is isolated.

2. The process as claimed in claim 1, wherein the liquid melamine is rapidly depressurized from an ammonia partial pressure p₁ between 400 and 200 bar.

3. The process as claimed in either of claims 1 or 2, wherein the liquid melamine is rapidly depressurized at a temperature which is 0 to 40°C above the melamine solidification point dependent on the respective ammonia partial pressure, but is below 350°C.

4. The process as claimed in claim 3, wherein the liquid melamine is rapidly depressurized at a temperature which is 0 to 20°C above the melamine solidification point dependent on the respective ammonia partial pressure, but is below 350°C.

5. The process as claimed in one of claims 1 to 4, wherein the liquid melamine is rapidly depressurized to an ammonia partial pressure p₂ between 175 and 6 bar, preferably 150 and 20 bar.

6. The process as claimed in one of claims 1 to 5, wherein the liquid melamine, before the rapid depressurization, is allowed to dwell at the prevailing ammonia partial pressure for 5 minutes to 20 hours, preferably 10 minutes to 10 hours, particularly preferably 30 minutes to 4 hours.

7. The process as claimed in one of claims 1 to 6, wherein, after the rapid depressurization, the solid melamine is allowed to dwell at the prevailing ammonia partial pressure and the prevailing temperature for 1 minute to 20 hours, preferably between 10 minutes and 10 hours, particularly preferably between 30 minutes and 3 hours.

8. The process as claimed in one of claims 1 to 7, wherein the solid melamine is allowed to dwell at a temperature below 290°C.

9. The process as claimed in one of claims 1 to 8, wherein the solid melamine is allowed to dwell at a temperature between 280 and 250°C.

10. The process as claimed in one of claims 1 to 9, wherein the depressurization is carried out in the vessel into which the liquid melamine was introduced.

11. The process as claimed in one of claims 1 to 10, wherein the depressurization is performed by transfer or spraying into one or more vessels.

12. The process as claimed in claim 11, wherein an ammonia atmosphere is present in this(these) vessel(s).

13. The process as claimed in either of claims 11 or 12, wherein roughly the same temperature prevails in the vessels as in the vessel from which depressurization is performed.

14. The process as claimed in one of claims 1 to 13, wherein the already solid melamine is cooled to room temperature by quenching with a cold liquid medium such as liquid ammonia, by mixing with cold gases, or by means of heat exchangers.

15. The process as claimed in one of claims 1 to 14, wherein a melamine to be purified that is already present as melt or liquid phase is brought to the temperature desired for the depressurization and to the ammonia partial pressure p₁ desired for the depressurization, in particular following a noncatalytic melamine synthesis at high pressure by reacting urea with ammonia.

16. The process as claimed in claim 15, wherein the molten or liquid melamine is brought to the desired temperature by lowering the temperature.

17. The process as claimed in claim 16, wherein the temperature is lowered at a cooling rate of 0.8 to 10°C/min.

18. The process as claimed in one of claims 15 to 17, wherein the NH₃/CO₂ gas mixture produced in the urea reaction is separated off from the liquid melamine prior to cooling, and the CO₂ dissolved in the liquid melamine is reduced by introducing gaseous ammonia.

19. The process as claimed in one of claims 1 to 14, wherein the melamine to be purified that is initially present as solid is brought to a temperature sufficient for reliable melting of the melamine, whereupon the melt is brought to the temperature desired for the depressurization and to the ammonia partial pressure p₁ desired for the depressurization.

20. The process as claimed in one of claims 1 to 19, wherein ammonia is fed until the respective desired temperature or desired pressure is set.

21. The process as claimed in one of claims 1 to 20, wherein the process is carried out continuously.

## Revendications

1. Procédé de fabrication de mélamine pure, **caractérisé en ce que**, à une température qui se situe de 0 - 60°C au-dessus du point fixe de la mélamine dépendant de la pression partielle d'ammoniac qui règne à chaque fois, tout en restant, toutefois, en dessous de 350°C, des pressions plus élevées permettant l'obtention d'une distance plus importante du point fixe de la mélamine que des pressions plus faibles, la mélamine liquide saturée d'ammoniac subit une détente rapide d'une pression partielle d'ammoniac p₁, comprise entre 400 et 100 bars, à une pression partielle d'ammoniac p₂, comprise entre 200 et 6 bars, la mélamine pure se déposant sous forme solide, à la suite de quoi l'on procède, dans une séquence quelconque, à la détente à la pression atmosphérique, au refroidissement à la température ambiante et à l'isolation de la mélamine pure.

2. Procédé selon la revendication 1, **caractérisé en ce que** la mélamine liquide subit une détente rapide d'une pression partielle d'ammoniac p₁ comprise entre 400 et 200 bars.

3. Procédé selon l'une quelconque des revendications 1 ou 2, **caractérisé en ce que** la mélamine liquide subit une détente rapide à une température qui se situe de 0 à 40°C au-dessus du point fixe de la mélamine dépendant à chaque fois de la pression partielle d'ammoniac, tout en restant, toutefois, en dessous de 350°C.

4. Procédé selon la revendication 3, **caractérisé en ce que** la mélamine liquide subit une détente rapide à une température qui se situe de 0 à 20°C au-dessus du point fixe de la mélamine dépendant à chaque fois de la pression partielle d'ammoniac, tout en restant, toutefois, en dessous de 350°C.

5. Procédé selon l'une quelconque des revendications 1 à 4, **caractérisé en ce que** la mélamine liquide subit une détente rapide à une pression partielle d'ammoniac p₂, comprise entre 175 et 6 bars, de préférence entre 150 et 20 bars.

6. Procédé selon l'une quelconque des revendications 1 à 5, **caractérisé en ce que** l'on laisse reposer la mélamine liquide, avant la détente rapide, pendant une durée de 5 minutes à 20 heures, de préférence de 10 minutes à 10 heures, particulièrement préférablement de 30 minutes à 4 heures, à la pression partielle d'ammoniac qui règne.

7. Procédé selon l'une quelconque des revendications 1 à 6, **caractérisé en ce que** l'on laisse reposer la mélamine solide, après la détente rapide, pendant une durée de 1 minute à 20 heures, de préférence comprise entre 10 minutes et 10 heures, particulièrement de préférence comprise entre 30 minutes et 3 heures, à la pression partielle d'ammoniac et à la température qui règnent.

8. Procédé selon l'une quelconque des revendications 1 à 7, **caractérisé en ce que** l'on laisse reposer la mélamine solide à une température inférieure à 290°C.

9. Procédé selon l'une quelconque des revendications 1 à 8, **caractérisé en ce que** l'on laisse reposer la mélamine solide à une température comprise entre 280 et 250°C.

10. Procédé selon l'une quelconque des revendications 1 à 9, **caractérisé en ce que** la détente se fait dans le récipient dans lequel la mélamine liquide a été introduite.

11. Procédé selon l'une quelconque des revendications 1 à 10, **caractérisé en ce que** la détente se fait par transfert ou par pulvérisation dans un ou plusieurs récipients.

12. Procédé selon la revendication 11, **caractérisé en ce qu'**une atmosphère d'ammoniac est présente dans ce ou dans ces récipients.

13. Procédé selon l'une quelconque des revendications 11 ou 12, **caractérisé en ce qu'**à peu près la même température règne dans les récipients que la température qui règne dans le récipient depuis lequel duquel on procède à la détente.

14. Procédé selon l'une quelconque des revendications 1 à 13, **caractérisé en ce que** la mélamine déjà solide est refroidie à la température ambiante par trempe à l'aide d'un milieu liquide froid, comme l'ammoniac liquide, par mélange avec des gaz froids ou par l'intermédiaire d'échangeurs thermiques.

15. Procédé selon l'une quelconque des revendications 1 à 14, **caractérisé en ce qu'**une mélamine à purifier, qui est déjà présente en tant que masse fondue ou phase liquide, en particulier à la suite d'une synthèse de mélamine non catalytique sous pression élevée par réaction de l'urée avec l'ammoniac, est amenée à la température souhaitée pour la détente et à la pression partielle d'ammoniac p₁ souhaitée pour la détente.

16. Procédé selon la revendication 15, **caractérisé en ce que** la mélamine fondue ou liquide est amenée à la température souhaitée par abaissement de la température.

17. Procédé selon la revendication 16, **caractérisé en ce que** la température est abaissée à une vitesse de refroidissement de 0,8 à 10°C/minute.

18. Procédé selon l'une quelconque des revendications 15 à 17, **caractérisé en ce que** le mélange gazeux NH₃/CO₂, se formant lors de la réaction de l'urée, est séparé, avant le refroidissement, de la mélamine liquide et **en ce que** le CO₂ dissous dans la mélamine liquide est réduit par l'introduction d'ammoniac gazeux.

19. Procédé selon l'une quelconque des revendications 1 à 14, **caractérisé en ce que** la mélamine à purifier, qui se présente tout d'abord sous forme solide, est amenée à une température suffisante pour la fusion certaine de la mélamine, à la suite de quoi la masse fondue est amenée à la température souhaitée pour la détente et à la pression partielle d'ammoniac p₁ souhaitée pour la détente.

20. Procédé selon l'une quelconque des revendications 1 à 19, **caractérisé en ce que** l'on introduit de l'ammoniac en vue du réglage de la température souhaitée ou de la pression d'ammoniac souhaitée à chaque fois.

21. Procédé selon l'une quelconque des revendications 1 à 20, **caractérisé en ce que** le procédé est exécuté en continu.
